(19) Europäisches Patentamt
European Patent Office
Office européen des brevets

(11) Veröffentlichungsnummer: **0 013 382**
**B1**

(12) # EUROPÄISCHE PATENTSCHRIFT

(45) Veröffentlichungstag der Patentschrift: **15.12.82**

(51) Int. Cl.³: **C 07 D 295/08**

(21) Anmeldenummer: **79105214.5**

(22) Anmeldetag: **17.12.79**

(54) **Verfahren zur Herstellung von m-Aminophenolen.**

(30) Priorität: **04.01.79 DE 2900193**

(43) Veröffentlichungstag der Anmeldung:
**23.07.80 Patentblatt 80/15**

(45) Bekanntmachung des Hinweises auf die Patenterteilung:
**15.12.82 Patentblatt 82/50**

(84) Benannte Vertragsstaaten:
**BE CH DE FR GB IT**

(56) Entgegenhaltungen:
**FR - A - 2 326 409**

**JOURNAL OF MEDICINAL CHEMISTRY, Band 13, Nr. 3, Mai 1970**
**E. F. ELSAGER: "Synthetic schistosomicides. XVI 5-(Mono- and dialkylamino)-2-nitrosophenols, 2-amino-5-(dialkylamino)phenols and related compounds", Seiten 370—376**

(73) Patentinhaber: **BASF Aktiengesellschaft**
**Carl-Bosch-Strasse 38**
**D-6700 Ludwigshafen (DE)**

(72) Erfinder: **Kolbinger, Hans Joergen, Dr. Dipl.-Chem.**
**Westring 60**
**D-6718 Gruenstadt 1 (DE)**
Erfinder: **Scheuermann, Horst, Dr. Dipl.-Chem.**
**Bexbacher Strasse 41**
**D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 013 382 B1

Verfahren zur Herstellung von m-Aminophenolen

Die Erfindung betrifft ein neues Verfahren zur Herstellung von m-Aminophenolen durch Umsetzung von Resorcin mit sekundären, cyclischen Aminen in Gegenwart von Phosphor-III-verbindungen in zwei Stufen unterschiedlicher Temperatur.

Es ist aus Houben-Weyl, Methoden der Organischen Chemie, Band 11/1, Seiten 160 bis 170, bekannt, daß man Phenole mit aliphatischen Aminen zu den entsprechenden Anilinverbindungen umsetzen kann. Wie Seite 162 näher angibt, wird die direkte Umsetzung auch im Falle reaktionsfähigerer Phenole wenig durchgeführt; man setzt in der Regel in Gegenwart wasserabspaltender bzw. katalytischer Zusätze, z.B. von Hydrochloriden der umzusetzenden Amine, Sulfaten, Phosphaten, Chloriden, Borsäure oder Jod, um. Die Ausbeute und Reinheit der Endstoffe sind bei diesen Verfahren unbefriedigend, so ziegen Umsetzungen von Resorcin mit verschiedenen Aminen in Gegenwart von Phosphorsäure nur Ergebnisse von 7 bis 16 Prozent und im Falle hoher Überschüsse an Amin 23 Prozent Ausbeute (Journal of Medicinal Chemistry (1970), Band 13, Seite 371 und 375). Insbesondere bilden sich teerähnliche und harzige Abscheidungen; daneben ist die Bildung von substituierten m-Phenylendiaminen nachteilig. Unter gewissen Reaktionsbedingungen, z.B. bei langer Reaktionsdauer, hoher Reaktionstemperatur und/oder in Gegenwart von Säuren bzw. Salzen als Katalysatoren, ist die Bildung dieser N-substituierten m-Phenylendiamine die Hauptreaktion. Die britische Patentschrift 168 689 zeigt, daß z.B. in Gegenwart des Sulfits oder Hydrochlorids des umzusetzenden Amins beide Hydroxylgruppen des Resorcins reagieren und nur in unwesentlichem Maße ein substituiertes m-Aminophenol erhalten wird.

Es ist aus der deutschen Offenlegungsschrift 24 26 186 bekannt, daß man am Stickstoffatom aliphatisch substituierte m-Aminophenole durch Umsetzung von Resorcin mit primären oder sekundären aliphatischen Aminen in Gegenwart einer Carbonsäure erhält. Bei diesem Verfahren wird das Ausgangsgemisch sofort auf die Reaktionstemperatur, im allgemeinen 150 bis 250°C, erhitzt und die einmal erreichte Temperatur 2 bis 12 Stunden beibehalten. In allen Beispielen wird während der gesamten Reaktionszeit eine spezifische Temperatur im Intervall von 200 bis 225°C eingehalten.

Die deutsche Offenlegungsschrift 26 06 363 beschreibt ein Verfahren zur Herstellung von Arylaminen durch Umsetzung von Monoalkoholen mit primären oder sekundären Arylaminen in Gegenwart von Phosphor-III-verbindungen. Auch bei diesem Verfahren, bei dem kein Dialkohol wie Resorcin verwendet wird, wird beschrieben (Seite 12, Absatz 3), daß die Umsetzung während der gesamten Reaktionszeit bei derselben Reaktionstemperatur gehalten wird. Beispiele 4 (240°C), 6, 10, 12, 13, 15 (230°C), 7 (210°C) und 14 (250°C) zeigen die Beibehaltung einer spezifischen Temperatur. In einer Variante der Arbeitsweise wird das Reaktionsgemisch kontinuierlich bis zur Endtemperatur erhitzt, wobei man auf die Ausgangstemperatur rasch aufheizt und dann langsam und kontinuierlich von der Ausgangstemperatur auf die Endtemperatur der Umsetzung erhitzt. So zeigen Beispiele 1 bis 3, 8 und 18 langsames Aufheizen von einer Anfangstemperatur im Intervall von 200 bis 211°C auf eine Endtemperatur im Intervall von 235 bis 250°C in 8 bis 15 Stunden, Beispiel 5 entsprechend von 177 auf 231°C (5 Stunden), Beispiele 9 und 17 entsprechend von 200 auf 223°C (9 Stunden), Beispiel 11 entsprechend von 206 auf 243°C (20 Stunden) und Beispiel 16 entsprechend von 184 auf 210°C (8 Stunden). Für jeden Ausgangsstoff wird in diesen Fällen eine spezifische Kombination von Aufheizzeit und Temperaturintervall der Erhitzung gewählt, ohne daß eine allgemeinere Regel bezüglich dieser Parameter und der Konstitution des Ausgangsstoffes festgestellt werden kann.

Bei den in den deutschen Offenlegungsschriften 25 49 957, 25 48 305 und 25 44 504 beschriebenen Verfahren werden Naphthole oder Phenole, in den beiden letzten Veröffentlichungen auch Resorcin, mit primären aromatischen Aminen in Gegenwart von Phosphor-III-verbindungen umgesetzt. Entsprechend dem Verfahren der deutschen Offenlegungsschrift 26 06 363 wird entweder ständig eine spezifische Reaktionstemperatur eingehalten oder während der Reaktion kontinuierlich von einer hohen Ausgangstemperatur auf eine höhere Endtemperatur erhitzt. Auch hier zeigen die Beispiele, daß für die Ausgangsstoffe unterschiedliche Ausgangs- und Endtemperaturen sowie Aufheizzeiten in Betracht kommen, ohne daß eine Zugehörigkeit spezifischer Parameter zu einer definierten Konstitution erkennbar wäre.

Alle diese Verfahren verwenden keine Heterocyclen als Ausgangsstoffe und geben keinen Hinweis auf deren Verhalten. Führt man Umsetzungen mit cyclischen Aminen beispielsweise nach der in der deutschen Offenlegungsschrift 26 06 363 beschriebenen Arbeitsweise durch, was bisher noch nicht beschrieben wurde, so erhält man unbefriedigende Ausbeuten an m-Aminophenolen bzw. eine hohe Ausbeute an Phenylendiaminen. Umsetzungen von Resorcin mit cyclischen Aminen erbringen nach Angaben von F. Effenberger et al., Chem. Ber., Band 103, Seiten 1 456 bis 1 462 (1970) auch in Abwesenheit von Katalysatoren unbefriedigende Ausbeuten, wobei im Falle von Morpholin und Piperidin Ausbeuten von weniger als 50 Prozent erhalten werden.

E. F. Elslager und D. F. Worth beschreiben in Journal of Medicinal Chemistry, Band 13, Seiten 370 bis 376 (1970) Versuche, in denen zur Herstellung von m-Aminophenolen Resorcin mit Pyrrolidin, Morpholin und Pyridin in Gegenwart von Phosphorsäure während 12 Stunden bei 200°C mit einer Ausbeute von 8, 16 bzw. 7 Prozent umgesetzt wurde.

Alle diese Verfahren sind mit Bezug auf die Herstellung von m-Aminophenolen mit Heterocyclen als Aminosubstituenten im Hinblick auf Ausbeute und Reinheit des Endstoffs unbefriedigend.

Es wurde nun gefunden, daß man m-Aminophenole der Formel

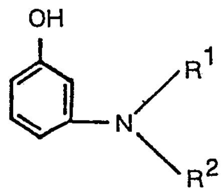

I,

worin $R^1$ und $R^2$ zusammen mit dem benachbarten Stickstoff atom Glieder eines heterocyclischen Restes bedeuten, durch Umsetzung von Resorcin mit Aminen in Gegenwart von Phosphorverbindungen, vorteilhaft erhält, wenn die Umsetzung von Resorcin mit sekundären cyclischen Aminen der Formel

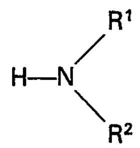

II,

worin $R^1$ und $R^2$ die vorgenannte Bedeutung besitzt, in Gegenwart von Phosphor-III-verbindungen der Formel

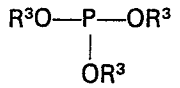

III,

worin die einzelnen Reste $R^3$ gleich oder verschieden sein können und jeweils ein Wasserstoffatom, einen aliphatischen, araliphatischen oder aromatischen Rest bedeuten, als Katalysatoren in einer Menge von 0,005 bis 0,5 Mol Katalysator je Mol Resorcin, in zwei Stufen unterschiedlicher Temperatur durchgeführt wird, wobei man mit

a) Ausgangsstoffen II, deren Reste $R^1$ und $R^2$ zusammen mit dem benachbarten Stickstoffatom Glieder eines 5-gliedrigen Ringes mit 4 Kohlenstoffatomen bedeuten, das Reaktionsgemisch in einer ersten Stufe binnen 10 bis 90 Minuten auf eine Temperatur im Bereich von 80 bis höchstens 130°C erhitzt, und das Reaktionsgemisch bei dieser gewählten ersten Stufentemperatur ohne wesentliches weiteres Erhitzen während mindestens 6 Stunden hält, in einer 2. Stufe dann binnen 10 bis 90 Minuten auf eine Temperatur im Bereich von oberhalb 130 bis 160°C aufheizt, und das Reaktionsgemisch bei dieser gewählten zweiten Stufentemperatur ohne wesentliches weiteres Erhitzen während mindestens einer Stunde hält oder

b) mit Ausgangsstoffen II, deren Reste $R^1$ und $R^2$ zusammen mit dem benachbarten Stickstoffatom Glieder eines heterocyclischen Restes mit mehreren Ringen oder mit einem Ring, der mehr als 4 Kohlenstoffatome und/oder mehr als ein Heteroatom enthält, bedeuten, das Reaktionsgemisch in einer ersten Stufe binnen 1 bis 3 Stunden auf eine Temperatur im Bereich von 150 bis höchstens 215°C erhitzt und das Reaktionsgemisch bei dieser gewählten ersten Stufentemperatur ohne wesentliches weiteres Erhitzen während mindestens 6 Stunden hält, in einer 2. Stufe dann binnen 10 bis 90 Minuten auf eine Temperatur im Bereich von oberhalb 215°C bis 240°C aufheizt und das Reaktionsgemisch bei dieser gewählten zweiten Stufentemperatur ohne wesentliches weiteres Erhitzen, während mindestens 2 Stunden hält.

Die Umsetzung kann für den Fall der Verwendung von Piperidin durch die folgenden Formeln wiedergegeben werden:

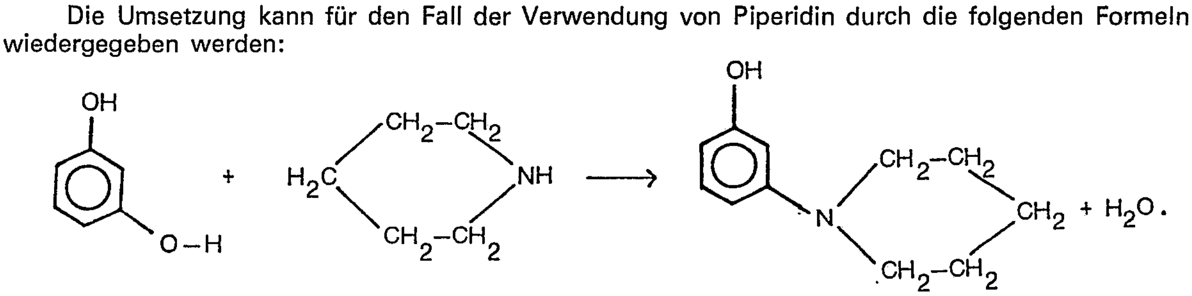

Im Vergleich zu den bekannten Verfahren liefert das Verfahren nach der Erfindung auf einfacherem und wirtschaftlicherem Wege m-Aminophenole, ausgehend von cyclischen Aminen, in besserer Ausbeute und Reinheit. Die Bildung von teerigen Rückständen bzw. N,N'-disubstituierten m-Phenylendiaminen ist überraschenderweise nicht in wesentlichem Maße zu beobachten. Das cyclische Amin kann in wirtschaftlicher Weise schon in etwa stöchiometrischer Menge verwendet werden. Im Vergleich zu Synthesen mit Phosphorsäure als Katalysator ist das erfindungsgemäße Verfahren selektiver, die Bildung heterogener Gemische ist nicht in wesentlichem Maße zu beobachten. Alle diese vorteilhaften Ergebnisse des erfindungsgemäßen Verfahrens sind überraschend. Auch hätte man vermuten müssen, daß zumindest wesentlich höhere Temperaturen, z.B. 250 bis 300°C, und weit längere Reaktionszeiten erforderlich wären, um befriedigende Ergebnisse zu erzielen.

Die Ausgangsstoffe werden in einem stöchiometrischen Verhältnis oder in einem Überschuß des einen oder anderen, vorzugsweise in einem Verhältnis von 1 bis 1,5, insbesondere 1 bis 1,2 Mol Ausgangsstoff II je Mol Resorcin, umgesetzt. Bevorzugte Ausgangsstoffe II und dementsphrechend bevorzugte Endstoffe I sind solche, in deren Formeln $R^1$ und $R^2$ zusammen mit dem benachbarten Stickstoffatom Glieder eines tricyclischen, vorzugsweise bicyclischen, insbesondere monocyclischen Amins bedeuten. Vorteilhaft liegen die Heteroatome nur in einem Ring der polycyclischen Amine. Im allgemeinen enthalten alle Heteroatome enthaltende Ringe nur eine Doppelbindung oder bevorzugt nur Einfachbindungen zwischen benachbarten Ringatomen. Bei polycyclischen und monocyclischen Aminen sind zwei Heteroatome, zweckmäßig ein Schwefelatom oder vorteilhaft ein Sauerstoffatom oder insbesondere ein Stickstoffatom, und insbesondere ein Heteroatom im Molekül und/oder 6-gliedrige und/oder 5-gliedrige Ringe im Molekül bevorzugt. Die genannten Reste und Ringe können noch durch unter den Reaktionsbedingungen inerte Gruppen, z.B. Alkylgruppen oder Alkoxygruppe mit 1 bis 4 Kohlenstoffatomen, substituiert sein.

Es sind z.B. folgende cyclische Amine als Ausgangsstoffe II geeignet: Für Ausführungsform a) Pyrrolidin, Δ2-Pyrrolin, Δ3-Pyrrolin; für Ausführungsform b) 4,5-Oxazolin, Oxazolidin, Isooxazolidin, Thiazolidin, Pyrazolidin, 3-Imidazolin, Piperidin, Morpholin, Tetrahydrothiazin, Piperazin, Alkylpiperazine mit 1 bis 4 Kohlenstoffatomen in der Alkylgruppe, insbesondere Methylpiperazin, Hexamethylenimin, Indolin, Isoindolin, 1,2,3,4-Tetrahydrochinolin, 1,2,3,4-Tetrahydroisochinolin. Bevorzugt sind Pyrrolidin, Piperidin, Morpholin.

Die Umsetzung wird im allgemeinen drucklos oder unter Druck, kontinuierlich oder diskontinuierlich durchgeführt. Vorteilhaft führt man die Reaktion ohne zusätzliches organisches Lösungsmittel durch; gegebenenfalls kommen aber auch unter den Reaktionsbedingungen inerte, organische Lösungsmittel, vorzugsweise mit Wasser nicht oder wenig mischbare Lösungsmittel, vorteilhaft aromatische Kohlenwasserstoff, z.B. Toluol, Äthylbenzol, o-, m-, p-Xylol, Isopropylbenzol, Methylnaphthalin; Äther, z.B. Äthylpropyläther, Diisobutyläther, Methyl-tert.-butyläther, n-Butyläthyläther, Di-n-butyläther, Di-iso-amyläther, Di-iso-propyläther, Anisol, Phenetol, Cyclohexylmethyläther, Diäthyläther; aliphatische oder cycloaliphatische Kohlenwasserstoffe, z.B. Heptan, Nonan, o-, m-, p-Cymol, Benzinfraktionen innerhalb eines Siedepunktintervalls von 70 bis 190°C, Cyclohexan, Methylcyclohexan, Dekalin, Hexan, Ligroin, 2,2,4-Trimethylpentan, 2,2,3-Trimethylpentan, 2,3,3-Trimethylpentan, Octan; und entsphrechende Gemische in Betracht. Zweckmäßig verwendet man das Lösungsmittel in einer Menge von 400 bis 10 000 Gewichtsprozent, vorzugsweise von 500 bis 3 000 Gewichtsprozent, bezogen auf Ausgangsstoff II.

Der Katalysator wird vorteilhaft in einer Menge von 0,008 bis 0,4, vorzugsweise von 0,01 bis 0,1 Mol Phosphor-III-verbindung III je Ausgangsstoff II verwendet. Bevorzugte Katalysatoren III sind solche, in deren Formel die einzelnen Reste $R^3$ gleich oder verschieden sein können und jeweils einen unsubstituierten Phenylrest oder Naphthylrest oder durch eine oder zwei Alkylgruppen mit jeweils 1 bis 4 Kohlenstoffatomen und/oder eine oder zwei Alkoxygruppen mit jeweils 1 bis 4 Kohlenstoffatomen substituierten Phenylrest oder Naphthylrest, ein Wasserstoffatomen, einen Alkylrest mit 1 bis 7 Kohlenstoffatomen oder einen Aralkylrest mit 7 bis 12 Kohlenstoffatomen bedeuten. Es kommen z.B. als Katalysatoren in Betracht: Tri-(2,6-Dimethyl)-phenylphosphit, Tri-(2-Methoxy)-phenylphosphit, Tri-o-tolylphosphit, Tri-m-tolylphosphit, Tri-p-tolylphosphit; Tri-o-xylylphosphite, Tri-m-xylylphosphite, Tri-p-xylylphosphite, wobei die Estergruppe vorteilhaft in m-Stellung zu einer der beiden Methylgruppen steht; Tri-$\alpha$-naphthylphosphit, Tri-$\beta$-naphthylphosphit, Triphenylphosphit; Tri-(methyl)-, Tri-(äthyl)-, Tri-(n-propyl)-, Tri-(isopropyl)-, Tri-(n-butyl)-, Tri-(isobutyl)-, Tri-(sek.-butyl)-, Tri-(n-pentyl)-, Tri-(n-heptyl)-, Trioctyl-, Tri-(n-hexyl)-, Tri-stearyl-, Tri-dodecyl-phosphit; Dimethyl-, Diäthyl-, Di-n-propyl-, Diisopropyl-, Di-n-butyl-, Diiso-butyl-, Di-sek.-butyl-, Di-n-pentyl-, Di-n-heptyl-, Di-octyl-, Di-n-hexyl-, Distearyl-, Didodecyl-phosphit; Methylphosphit, Äthylhosphit, Propylphosphit, Isopropylphosphit, Butylphosphit, Isobutylphosphit, sek.-Butylphosphit, n-Pentyl-, n-Heptyl-, Octyl-, n-Hexyl-phosphit, Stearylphosphit, Dodecylphosphit; phosphorige Säure; Tri-benzyl-, Triphenyläthyl-, Triphenylpropyl-phosphit, Di-benzyl-, Diphenyläthyl-, Diphenylpropyl-phosphit, Benzyl-phosphit, Phenyläthylphosphit, Phenylpropylphosphit; Di-(2,6-Dimethyl)-phenylphosphit, Di-(2-Methoxy)-phenylphosphit, Di-o-tolylphosphit, Di-m-tolylphosphit, Di-p-tolylphosphit; Di-o-xylylphosphite, Di-m-xylylphosphite, Di-p-xylylphosphite, wobei die Estergruppe vorteilhaft in m-Stellung zu einer der beiden Methylgruppen steht, Di-$\alpha$-naphthylphosphit, Di-$\beta$-naphthylphosphit, Diphenylphosphit; 2,6-Dimethylphenylphosphit, 2-Methoxyphenylphosphit, o-Tolylphosphit, m-Tolylphosphit, p-Tolylphosphit, o-Xylylphosphite, m-Xylylphosphite, p-Xylylphosphite,

wobei die Estergruppe vorteilhaft in m-Stellung zu einer der beiden Methylgruppen steht, α-Naphthylphosphit, β-Naphthylphosphit, Phenylphosphit; entsprechende Phosphite mit 3 vorgenannten, aber völlig oder teilweise unterschiedlichen Resten, z.B. Phenyldimethylphosphit, Methyldiäthylphosphit, Methyläthylpropylphosphit, Diphenylbutylphosphit, Phenyldiäthylphosphit, Äthylphenylphosphit, Butylphenylphosphit, Benzyldiäthylphosphit.

Bevorzugt sind: Tri-n-butyl-, Tribenzyl-phosphit, phosphorige Säure, Dibutylphosphit, Diisopropylphosphit, Dibenzylphosphit, Triisopropylphosphit, Distearylphosphit, Didodecylphosphit, Triphenylphosphit, Tritolyphosphit, Trixylylphosphite, Trinaphthylphosphit, Tri-n-hexylphosphit, Diphenylphosphit, Di-n-hexylphosphit, Benzyldiäthylphosphit.

Die erfindung geht von der Beobachtung aus, daß nicht spezifische Anfangs- und Endtemperaturen einer stetigen Erhitzungsoperation für die Umsetzung cyclischer Amine eine entscheidende Rolle spielen. Vielmehr ist es ein entscheidendes erfinderisches Merkmal, daß das Gemisch der Reaktionskomponenten in 2 Stufen umgesetzt wird und in jeder Stufe rasch auf die jeweilige Stufentemperatur erhitzt und daraufhin längere Zeit diese Stufentemperatur gehalten wird. Im Falle der Gruppe a) der Ausgangsstoffe II, insbesondere von Pyrrolinen und Pyrrolidin heizt man a 1) das Ausgangsgemisch zweckmäßig binnen 10 bis 90, insbesondere 30 bis 60 Minuten auf eine Temperatur von 80 bis höchstens 130°C, vorzugsweise von 90 bis 120°C, und hält das Reaktionsgemisch bei dieser gewählten ersten Stufentemperatur ohne wesentliches weiteres Erhitzen, zweckmäßig mit einer Schwankungsbreite von ±3°C, während mindestens 6 Stunden, zweckmäßig von 6 bis 20, vorzugsweise 6 bis 15, insbesondere 7 bis 12 Stunden, heizt a 2) dann zweckmäßig binnen 10 bis 90, insbesondere von 30 bis 60 Minuten auf eine Temperatur von oberhalb 130 bis 160°C, vorzugsweise von 140 bis 155°C und hält das Reaktionsgemisch bei dieser gewählten zweiten Stufentemperatur ohne wesentliches weiteres Erhitzen, zweckmäßig mit einer Schwankungsbreite von ±3°C, während mindestens einer Stunde, zweckmäßig von 1 bis 9 Stunden, vorzugsweise 2 bis 5, insbesondere 2 bis 4 Stunden. Im Falle der Gruppe b) der Ausgangsstoffe II, insbesondere von Piperidin und Morpholin, heizt man b 1) das Ausgangsgemisch zweckmäßig binnen 1 bis 3, insbesondere 1 bis 2 Stunden auf eine Temperatur von 150 bis höchstens 215°C, vorzugsweise von 170 bis 210°C, insebesondere 180 bis 210°C und hält das Reaktionsgemisch bei dieser gewählten ersten Stufentemperatur ohne wesentliches weiteres Erhitzen, zweckmäßig mit einer Schwankungsbreite von ±3°C, während mindestens 6 Stunden, zweckmäßig von 6 bis 20, vorzugsweise 6 bis 15, insbesondere 6 bis 12 Stunden, heizt b 2) dann zweckmäßig binnen 10 bis 90, insbesondere 30 bis 70 Minuten auf eine Temperatur von oberhalb 215°C bis 240°C, vorzugsweise 220 bis 235, insbesondere von 220 bis 230°C und hält das Reaktionsgemisch bei dieser gewählten zweiten Stufentemperatur ohne wesentliches weiteres Erhitzen, zweckmäßig mit einer Schwankungsbreite von ±3°C, während mindestens 2 Stunden, zweckmäßig 2 bis 9, insbesondere 2,8 bis 7 Stunden.

Die Reaktion kann wie folgt durchgeführt werden: Ein Gemisch von Katalysator III, Ausgangsstoff II und Resorcin und gegebenenfalls des Lösungsmittels wird während vorgenannten Reaktionszeiten und zwei Reaktionstemperaturen umgesetzt. Aus dem Gemisch wird dann der Endstoff in üblicher Weise, z.B. durch Lösen in Säure, Filtration und Fällen des Filtrats mit z.B. Sodalösung, isoliert. Zur Aufarbeitung kann man auch die Schmelze des Reaktionsgemischs mit heißem Wasser behandeln und den Endstoff nach dem Erkalten abfiltrieren.

Die nach dem Verfahren der Erfindung herstellbaren m-Aminophenole I sind wertvolle Zwischenprodukte für die Herstellung von Pflanzenschutzmitteln, von optischen Aufhellern, insbesondere von Amino-Cumarin-Derivaten, und von Farbstoffen, besonders der Xanthen-, Pyronin-, Rhodamin- und Oxazin-Reihen. Bezüglich der Verwendung wird auf die genennten Veröffentlichungen, Ullmans Encyklopädie der technischen Chemie, Band 3, Seite 473 und Kirk-Othmer, Encylopedia of Chemical Technology, Band 2, Seiten 213 ff (1963), verwiesen.

Die im folgenden aufgeführten Teile sind Gewichtsteile.

## Beispiel 1

770 Teile Resorcin, 653 Teile Morpholin und 41 Teile 50-gewichtsprozentige phosphorige Säure werden im Druckautoklaven 12 Stunden bei 210°C und weitere 6 Stunden bei 230°C gerührt, wobei ein Innendruck von 20 bar entsteht. Die Aufheizzeiten für beide Stufen betragen jeweils 1,1 Stunden. Nach dem Abkühlen und Entspannen läßt man bei 80°C 1 500 Teile Wasser gleicher Temperatur zulaufen und rührt, bis Raumtemperatur erreicht ist. Nach Absaugen, Waschen und Trocknen im Vakuum bei 50°C erhält man 1 078 Teile (gaschromatographisch bestimmt) 3-Morpholinyl-(N)-phenyol (86% der Theorie) vom Fp 121 bis 125°C.

## Beispiel 2

220 Teile Resorcin, 187 Teile Piperidin und 8 Teile 50-gewichtsprozentige phosphorige Säure werden im Druckautoklaven 9 Stunden bei 181°C und 3 Stunden bei 223°C gerührt. Die Aufheizzeiten für beide Stufen betragen jeweils 1,1 Stunden. Man löst das Gemisch mittels Sodalösung. Man erhält 289 Teile (gaschromatographisch bestimmt) 3-Piperidinylphenol (84% der Theorie) vom Fp 114 bis 116°C.

Beispiel 3

660 Teile Resorcin, 448 Teile Pyrrolidin und 72 Teile 50-gewichtsprozentige phosphorige Säure werden im Druckautoklaven 9 Stunden bei 100°C und 2,1 Stunden bei 150°C gerührt, wobei ein Eigendruck von 12 bar entsteht. Die Aufheizzeiten für beide Stufen betragen jeweils 0,7 Stunden. Nach dem Entspannen belüftet man mit Stickstoff, läßt bei 100°C 2 000 Teile heißes Wasser zulaufen und rührt, bis Raumtemperatur erreicht ist. Nach Absaugen, Waschen und Trocknen im Vakuum bei 50°C erhält man 923 Teile 3-Pyrrolidinyl-(N)-phenol vom Schmelzpunkt 123 bis 127°C. Die gaschromatographisch ermittelte Reinheit liegt bei 97 Prozent, die Ausbeute bei 86% der Theorie.

Beispiel 4

660 Teile Resorcin, 590 Teile Morpholin und 33 Teile Triphenylphosphit werden im Druckautoklaven 12 Stunden bei 210°C und weitere 6 Stunden bei 235°C gerührt, wobei ein Innendruck von 23 bar entsteht. Die Aufheizzeiten für beide Stufen betragen jeweils 1,1 Stunden. Nach dem Abkühlen und Entspannen läßt man bei 80°C 800 Teile Wasser gleicher Temperatur zulaufen und rährt, bis Raumtemperatur erreicht ist. Nach Absaugen, Waschen und Trocknen im Vakuum bei 50°C erhält man 913 Teile (gaschromatographisch bestimmt) 3-Morpholinyl-(N)-phenol (85% der Theorie) vom Fp 121 bis 125°C.

**Patentanspruch**

Verfahren zur Herstellung von m-Aminophenolen der Formel

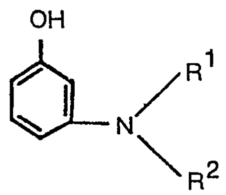

I,

worin $R^1$ und $R^2$ zusammen mit dem benachbarten Stickstoff Glieder eines heterocyclischen Restes bedeuten, durch Umsetzung von Resorcin mit Aminen in Gegenwart von Phosphorverbindungen, dadurch gekennzeichnet, daß die Umsetzung von Resorcin mit sekundären cyclischen Aminen der Formel

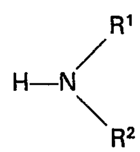

II,

worin $R^1$ und $R^2$ die vorgenannte Bedeutung besitzen, in Gegenwart von Phosphor-III-verbindungen der Formel

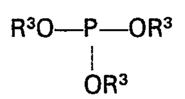

III,

worin die einzelnen Reste $R^3$ gleich oder verschieden sein können und jeweils ein Wasserstoffatom, einen aliphatischen, araliphatischen oder aromatischen Rest bedeuten, als Katalysatoren in einer Menge von 0,005 bis 0,5 Mol Katalysator je Mol Resorcin, in zwei Stufen unterschiedlicher Temperatur durchgeführt wird, wobei man mit

a) Ausgangsstoffen II, deren Reste $R^1$ und $R^2$ zusammen mit dem benachbarten Stickstoffatom Glieder eines 5-gliedrigen Ringes mit 4 Kohlenstoffatomen bedeuten, das Reaktionsgemisch in einer ersten Stufe binnen 10 bis 90 Minuten auf eine Temperatur im Bereich von 80 bis höchstens 130°C erhitzt, und das Reaktionsgemisch bei dieser gewählten ersten Stufentemperatur ohne wesentliches weiteres Erhitzen während mindestens 6 Stunden hält, in einer 2. Stufe dann binnen 10 bis 90 Minuten auf eine Temperatur im Bereich von oberhalb 130 bis 160°C aufheizt, und das Reaktionsgemisch bei dieser gewählten zweiten Stufentemperatur ohne wesentliches weiteres Erhitzen während mindestens einer Stunde hält oder

b) mit Ausgangsstoffen II, deren Reste $R^1$ und $R^2$ zusammen mit dem benachbarten Stickstoffatom Glieder eines heterocyclischen Restes mit mehreren Ringen oder mit einem Ring, der mehr als 4 Kohlenstoffatome und/oder mehr als ein Heteroatom enthält, bedeuten, das Reaktionsgemisch in

einer ersten Stufe binnen 1 bis 3 Stunden auf eine Temperatur im Bereich von 150 bis höchstens 215°C erhitzt und das Reaktionsgemisch bei dieser gewählten ersten Stufentemperatur ohne wesentliches weiteres Erhitzen während mindestens 6 Stunden hält, in einer 2. Stufe dann binnen 10 bis 90 Minuten auf eine Temperatur im Bereich von oberhalb 215°C bis 240°C aufheizt und das Reaktionsgemisch bei dieser gewählten zweiten Stufentemperatur ohne wesentliches weiteres Erhitzen, während mindestens 2 Stunden hält.

**Revendication**

Procédé pour la préparation de m-aminophénols de formule

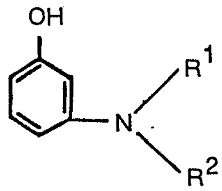

(I)

dans laquelle $R^1$ et $R^2$ représentent, avec l'atome d'azote voisin, des termes d'un radical hétérocyclique, par réaction de résorcine avec des amines en présence de composés du phosphore, caractérisé en ce que la réaction de la résorcine avec des amines cycliques secondaires de formule

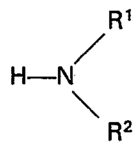

(II)

dans laquelle $R^1$ et $R^2$ ont les significations données ci-dessus, est menée en présence de composés du phosphore trivalent de formule

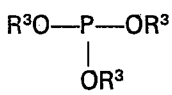

(III)

dans laquelle les différents radicaux $R^3$ peuvent être semblables ou différents et représentent chacun un atome d'hydrogène, un radical aliphatique, araliphatique ou aromatique, en tant que catalyseurs introduits dans une proportion de 0,005 à 0,5 moles de catalyseur par mole de résorcine, en deux stades de températures différentes, en procédant de la manière suivante:

a) avec des substances de départ II dont les radicaux $R^1$ et $R^2$ représentent, avec l'atome d'azote voisin, des termes d'un noyau hexagonal à 4 atomes de carbone, on chauffe le mélange réactionnel, en un premier stade, en l'espace de 10 à 90 minutes, jusqu'à une température dans la gamme de 80 à 130°C au maximum et on maintient le mélange réactionnel à cette température choisie pour le premier stade sans autre chauffage appréciable pendant 6 heures au moins, puis, en un second stade, on chauffe, en l'espace de 10 à 90 minutes, jusqu'à une température dans la gamme de plus de 130 à 160°C et on maintient le mélange réactionnel à cette température choisie pour le second stade, sans autre chauffage appréciable, pendant une heure au moins; ou

b) avec des substances de départ II dont les radicaux $R^1$ et $R^2$ représentent, avec l'atome d'azote voisin, des termes d'un radical hétérocyclique comportant plusieurs noyaux ou un noyau qui contient plus de 4 atomes de carbone et/ou plus d'un hétéroatome, on chauffe le mélange réactionnel, en un premier stade, en l'espace de 1 à 3 heures, jusqu'à une température dans la gamme de 150 à 215°C au maximim et on maintient le mélange réactionnel à cette température choisie pour le premier stade, sans autre chauffage appréciable pendant 6 heures au moins, puis, en un second stade, on chauffe en l'espace de 10 à 90 minutes jusqu'à une température dans la gamme de plus de 215°C à 240°C et on maintient la mélange réactionnel à cette température choisie pour le second stade, sans autre chauffage appréciable, pendant 2 heures au moins.

**Claim**

Process for the preparation of m-aminophenols of the formula

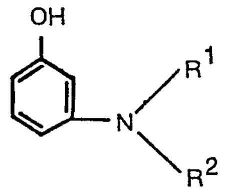

I

where $R^1$ and $R^2$ together with the adjoining nitrogen atom denote members of a heterocyclic radical, by reacting resorcinol with amines in the presence of phosphorus compounds, wherein the reaction of resorcinol with secondary cyclic amines of the formula

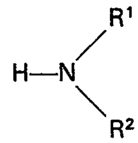

II

where $R^1$ and $R^2$ have the above meaning, is carried out in the presence of phosphorus-III compounds of the formula

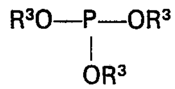

III

where the individual radicals $R^3$ may be identical or different and each denotes a hydrogen atom, or an aliphatic, araliphatic or aromatic radical, as catalysts, in an amount of 0.005 to 0.5 mole of catalyst per mole of resorcinol, in two stages at different temperatures, and

a) in the case of starting materials II, whose radicals $R^1$ and $R^2$ together with the adjacent nitrogen atom denote members of a 5-membered ring having 4 carbon atoms, the reaction mixture is heated, in a first stage, to a temperature in the range of from 80 to at most 130°C in the course of from 10 to 90 minutes and is kept for at least 6 hours at this chosen first-step temperature, without significant additional heating, and, in a 2nd stage, is then heated in the course of from 10 to 90 minutes to a temperature in the range of from above 130 to 160°C and kept at this chosen second-step temperature for at least one hour without significant additional heating, or

b) in the case of starting materials II, whose radicals $R^1$ and $R^2$ together with the adjacent nitrogen atom denote members of a heterocyclic radical possessing a plurality of rings or possessing one ring which contains more than 4 carbon atoms and/or more than one hetero-atom, the reaction mixture is heated, in a first stage, to a temperature in the range of from 150 to at most 215°C in the course of from 1 to 3 hours and is kept for at least 6 hours at this chosen first-step temperature, without significant additional heating, and, in a 2nd stage, is then heated in the course of from 10 to 90 minutes to a temperature in the range of from above 215°C to 240°C and kept at this chosen second-step temperature for at least 2 hours without significant additional heating.